# EUROPEAN PATENT SPECIFICATION

(11) **EP 4 032 557 B1**
(45) Date of publication and mention of the grant of the patent: **25.12.2024**
(21) Application number: 20865810.4
(22) Date of filing: 10.09.2020
(51) Int. Cl.: A61L 15/32, A61L 24/00, A61L 24/10, A61P 5/00, A61P 31/04, A61P 35/00

(54) **BIOLOGICAL TISSUE ADHESIVE SHEET , BIOLOGICAL TISSUE REINFORCING MATERIAL KIT, AND METHOD FOR PRODUCING BIOLOGICAL TISSUE ADHESIVE SHEET**
KLEBENDE BIOLOGISCHE GEWEBEFOLIE, KIT FÜR BIOLOGISCHES GEWEBEVERSTÄRKUNGSMATERIAL UND VERFAHREN ZUM PRODUZIEREN EINER KLEBENDEN BIOLOGISCHEN GEWEBEFOLIE
FEUILLE ADHÉSIVE DE TISSU BIOLOGIQUE, KIT DE MATÉRIAU DE RENFORCEMENT DE TISSU BIOLOGIQUE ET PROCÉDÉ DE PRODUCTION DE FEUILLE ADHÉSIVE DE TISSU BIOLOGIQUE

(30) Priority: 18.09.2019 JP 2019168904
(43) Date of publication of application: 27.07.2022
(73) Proprietor: National Institute for Materials Science, Tsukuba-shi, Ibaraki 305-0047 (JP)
(72) Inventor: TAGUCHI, Tetsushi, Tsukuba-shi, Ibaraki 305-0047 (JP)
(74) Representative: Dehns
(86) International application number: PCT/JP2020/034213
(87) International publication number: WO 2021/054233

(56) References cited:
- EP-A1- 1 319 415
- EP-A1- 3 862 016
- WO-A1-2014/112208
- WO-A1-2017/126390
- JP-A- 2000 037 450
- JP-A- 2003 301 362
- JP-A- 2003 301 362
- JP-A- 2004 321 484
- JP-A- 2020 141 190
- AKIRA ISOGAI: "Nanoscale TEMPO-oxidized cellulose nanofibers", 1 January 2011 (2011-01-01), XP093167261
- RYO MIZUTA ET AL: "Enhanced Sealing by Hydrophobic Modification of Alaska Pollock-Derived Gelatin-Based Surgical Sealants for the Treatment of Pulmonary Air Leaks", MACROMOLECULAR BIOSCIENCE, vol. 17, no. 4, 15 November 2016 (2016-11-15), DE, pages 1600349, XP055581004, ISSN: 1616-5187, DOI: 10.1002/mabi.201600349
- YAMAOKA, MASATOSHI ET AL.: "Novel Alaska Pollock Gelatin Sealant Shows High Adhesive Quality and Conformability", ANN. THORAC. SURG., vol. 107, June 2019 (2019-06-01), pages 1656 - 1662, XP085694779, ISSN: 0003-4975, DOI: 10.1016/j.athoracsur.2018.11.074
- TAGUCHI, TETSUSHI: "Development of high strength and biocompatible tissue adhesive for medical application", JOURNAL OF JAPANESE SOCIETY FOR BIOMATERIALS, vol. 36, no. 4, 1 January 2018 (2018-01-01), JP , pages 298 - 301, XP009534863, ISSN: 1347-7080
- HIROAKI ICHIMARU, YOSUKE MIZUNO, CHEN XI, AKIHIRO NISHIGUCHI, TETSUSHI TAGUCHI: "P67A Development of a hydrophobically modified Alaska pollock-derived gelatin sheet possessing sealing effect for pulmonary air leaks", LECTURE ABSTRACTS OF THE 58TH SYMPOSIUM OF THE ADHESION SOCIETY OF JAPAN; JUNE 18-19, 2020, vol. 58, 18 June 2020 (2020-06-18) - 19 June 2020 (2020-06-19), JP, pages P67A, XP009534700

## Description

### Technical Field

The present invention relates to a biological tissue adhesive sheet, a biological tissue reinforcement material kit, and a method for producing a biological tissue adhesive sheet.

### Background Art

In medical fields such as thoracic surgery, gastroenterological surgery, and gastroenterological medicine, sheet-shaped medical devices (medical sheets) are used for the purpose of reinforcing sutures after surgery, preventing air leakage, and the like.

Patent Literature 1 describes, as such a medical sheet, "A suture prosthetic material comprising, in at least a part thereof, a nonwoven fabric that is made of polyglycolic acid and is produced by melt blowing".

Patent Literature 2 was published after the priority date of the present case and is thus Article 54(3) EPC prior art. Patent literature 2 discloses an angiogenesis promoter capable of exerting an angiogenesis promoting effect without containing any growth factor. The angiogenesis promoter comprises at least one selected from the group consisting of a gelatin derivative and a crosslinked product of the gelatin derivative as an active ingredient.

Non-Patent Literature 1 discloses cross-linked gelatin derived from Alaskan pollock and its use as surgical sealant having enhanced adhesion strength.

### Citation List

### Patent Literature

Patent Literature 1: JP 2000-157622 A
Patent Literature 2: EP 3 862 016 A1

### Non-patent Literature

Non-patent Literature 1: Ryo Mizuta ET AL: "Enhanced Sealing by Hydrophobic Modification of Alaska Pollock-Derived Gelatin-Based Surgical Sealants for the Treatment of Pulmonary Air Leaks", Macromolecular Bioscience, vol. 17, no. 4, 15 November 2016 (2016-11-15), page 1600349, XP055581004, DE ISSN: 1616-5187, DOI: 10.1002/mabi.201600349.

### Summary of Invention

### Technical Problem

The present inventor and his colleagues have found that it may be difficult to have, with use of the suture prosthetic material described in Patent Literature 1, sufficient tissue adhesiveness in a wet environment at a biological tissue surface.

Therefore, in view of the above-mentioned circumstances, an object of the present invention is to provide a biological tissue adhesive sheet having excellent tissue adhesiveness.

Another object of the present invention is to provide a biological tissue reinforcement material kit and a method for producing a biological tissue adhesive sheet.

### Solution to Problem

As a result of intensive studies focusing on gelatins having excellent biocompatibility, the present inventor has found that the above-mentioned objects can be achieved by the following configuration.

The present invention provides a biological tissue adhesive sheet comprising a nonwoven fabric made of fibers containing a crosslinked cold-water fish gelatin, wherein the cold-water fish gelatin is at least one selected from the group consisting of cod gelatin, sea bream gelatin, salmon gelatin, and a genetically-engineered gelatin derived from cod, sea bream, or salmon, and wherein the nonwoven fabric has a thickness in a range from 100 µm to 500 µm.

The biological tissue adhesive sheet may contain an agent fixed to the nonwoven fabric.

The agent may be an anticancer agent.

The agent may an antimicrobial agent.

The agent may be a growth factor.

The present invention also provides a biological tissue reinforcement material kit comprising the biological tissue adhesive sheet as described herein, and an instruction describing how to use the biological tissue adhesive sheet and/or precautions for use.

The present invention also provides a method for producing the biological tissue adhesive sheet as described herein, the method comprising:
preparing a composition by adding a cold-water fish gelatin and optionally a crosslinking agent and/or an agent to a solvent, the cold-water fish gelatin being at least one selected from the group consisting of cod gelatin, sea bream gelatin, salmon gelatin, and a genetically-engineered gelatin derived from cod, sea bream, or salmon,
electrospinning the composition to prepare a nonwoven fabric made of fibers containing the cold-water fish gelatin; and
applying energy to the nonwoven fabric to produce a biological tissue adhesive sheet;
wherein the nonwoven fabric has a thickness in a range from 100 µm to 500 µm.

The method may further include irradiating the biological tissue adhesive sheet with an ultraviolet ray to hydrophilize surfaces of the fibers containing the cold-water fish gelatin.

The biological tissue adhesive sheet may be irradiated with the ultraviolet ray for 0.5 minutes to 30 minutes.

### Advantageous Effects of Invention

According to the present invention, it is possible to provide a biological tissue adhesive sheet having excellent tissue adhesiveness. Moreover, in a predetermined burst strength test, the biological tissue adhesive sheet of the present invention exhibits a higher burst strength than a conventional nonwoven fabric made of a biodegradable absorbent material does.

Further, according to the present invention, it is also possible to provide a biological tissue reinforcement material kit and a method for producing a biological tissue adhesive sheet.

Since the biological tissue adhesive sheet of the present invention has a configuration including a nonwoven fabric made of fibers containing a crosslinked cold-water fish gelatin, when the biological tissue adhesive sheet contains an agent (for example, an anticancer agent) fixed to the nonwoven fabric, the sheet can be used not only as a tissue reinforcement material but also as an agent eluting sheet.

### Brief Description of Drawings

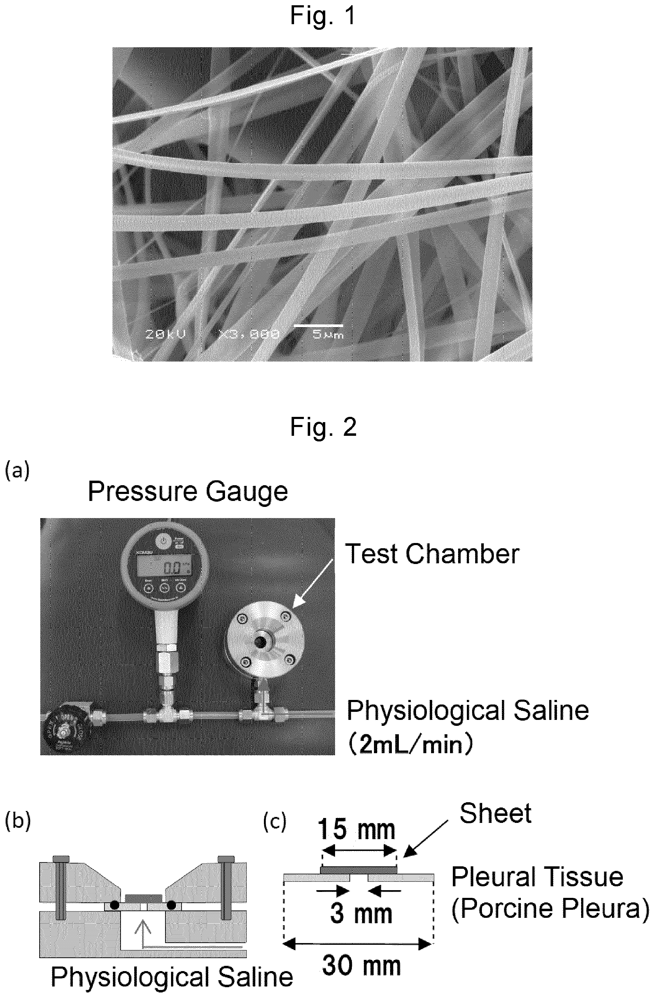

Fig. 1 shows a SEM image of a cod gelatin fiber sheet of Example.
Fig. 2 shows an outline of a burst strength test in the section of Examples; Fig. 2(a) is a photograph showing a configuration of an apparatus used in the test, Fig. 2(b) is a schematic view showing a cross section of a test chamber, and Fig. 2(c) is a schematic view showing a configuration of an adherend (porcine pleura) and a cod gelatin fiber sheet.

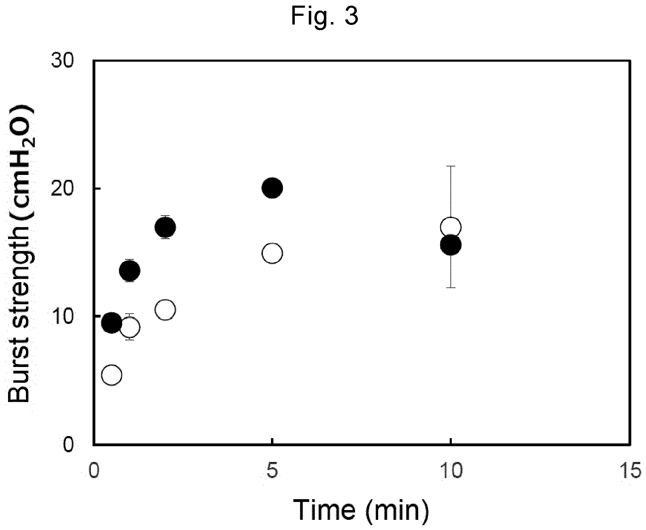

Fig. 3 is a graph showing a relation between the standing time (min) and the burst strength (cmH₂O) of a cod gelatin fiber sheet (AdFS1) that was prepared with thermal crosslinking for 1 hour, and a cod gelatin fiber sheet (UV-AdFS1) that was AdFS1 irradiated with ultraviolet rays for 60 minutes in a burst strength test on a porcine pleura. Filled circles: UV-AdFS1, open circles: AdFS1

### Description of Embodiments

Hereinafter, embodiments of the present invention will be described.

### [Biological Tissue Adhesive Sheet]

The biological tissue adhesive sheet according to an embodiment of the present invention includes a nonwoven fabric made of fibers containing a crosslinked cold-water fish gelatin.

Hereinafter, the biological tissue adhesive sheet according to the present embodiment, which includes a nonwoven fabric made of fibers containing a crosslinked cold-water fish gelatin, is sometimes referred to as a "cold-water fish gelatin fiber sheet".

As used herein, the term "cold-water fish gelatin" refers to a gelatin derived from a cold-water fish.

It is known that habitat of cold-water fishes has a low water temperature (about 10°C to 15°C as a standard). Examples of the cold-water fish include, but are not limited to, cod, sea bream, and salmon. Here, the term "cod" is a generic name of fishes belonging to Gadinae, and examples thereof include Pacific cod, Saffron cod, and Alaska pollock. The term "sea bream" is a generic name of fishes belonging to Sparidae, and examples thereof include red sea bream, black sea bream, and yellowback sea bream. The term "salmon" is a generic name of fishes belonging to Salmonidae, and examples thereof include chum salmon, Atlantic salmon, and sockeye salmon.

The cold-water fish may be a natural fish or a genetically-engineered fish.

In a preferred embodiment, the cold-water fish gelatin is a gelatin derived from cod (cod gelatin). A gelatin derived from Alaska pollock is more preferred.

The cold-water fish gelatin may be produced from raw material collagen (collagen of a cold-water fish) that is pretreated with an acid (inorganic acid such as hydrochloric acid or sulfuric acid) or an alkali (lime). The pretreatment on the raw material collagen affects the hydrolysis of acid amide (that is, deamidation of amino acid side chains) in the collagen that occurs in the process of producing a gelatin. Therefore, the isoionic point of the gelatin varies depending on the presence or absence and conditions of the pretreatment. Specifically, the former acid-treated gelatin has an isoionic point of about pH 8 to 9, and the latter alkali-treated gelatin has an isoionic point of about pH 5.

The cold-water fish gelatin has an amino acid sequence similar to that of a gelatin derived from a mammal, is easily degraded by an enzyme, and has high biocompatibility. At the same time, the cold-water fish gelatin has a lower freezing point than that of a gelatin derived from a mammal, and has normal temperature fluidity. This is because the number (content) of hydroxyproline residues in the collagen of cold-water fishes is smaller than that in the collagen of mammals, and thus the collagen of cold-water fishes has a lower denaturation temperature. In addition, among fishes that are heterothermic animals, the denaturation temperature of collagen varies depending on the environment in which fishes are grown. Therefore, a fiber sheet containing a cold-water fish gelatin has a feature that the sheet is easily hydrated and gelates in vivo.

Advantages achieved when the biological tissue adhesive sheet of the present embodiment is a cold-water fish gelatin fiber sheet include physical properties of the cold-water fish gelatin as described above. Therefore, it is considered more effective to select the kind of the cold-water fish gelatin according to the intended use, application site, and the like of the biological tissue adhesive sheet.

The molecular weight of the cold-water fish gelatin is not particularly limited, but is preferably in the range of 20,000 to 150,000, and more preferably in the range of 30,000 to 100,000. When the molecular weight is within the above-mentioned range, a biological tissue adhesive sheet having even better tissue adhesiveness is obtained, the sheet itself is excellent in strength determined by a predetermined burst strength test as described in the section of Examples described later (hereinafter, the strength is also referred to as "film strength"), and the sheet has good handleability. As used herein, the term "molecular weight of the cold-water fish gelatin" means the weight average molecular weight (Mw) in terms of standard pullulan measured by gel permeation chromatography (GPC).

The cold-water fish gelatin may be a gelatin reduced in endotoxin content by a conventionally known method.

### <Nonwoven Fabric>

In the biological tissue adhesive sheet of the present embodiment, the nonwoven fabric is made of fibers containing a crosslinked cold-water fish gelatin (hereinafter, the fibers are also referred to as "cold-water fish gelatin fibers").

As used herein, the term "crosslinked cold-water fish gelatin" means a crosslinked product of a cold-water fish gelatin. The crosslinked product of a cold-water fish gelatin typically means a reaction product that is obtained by applying energy such as heat or light to the cold-water fish gelatin and/or reacting the cold-water fish gelatin with a crosslinking agent.

As used herein, the term "crosslinked cold-water fish gelatin" or "crosslinked product of a cold-water fish gelatin" means a cold-water fish gelatin in a state of being crosslinked by an irreversible crosslinking reaction, that is, a crosslinked product of a cold-water fish gelatin that is obtained by an irreversible crosslinking reaction, and does not encompass a cold-water fish gelatin having a crosslinked structure (physical crosslinked structure) formed by an intermolecular and/or intramolecular interaction of the cold-water fish gelatin.

The method of applying energy to the cold-water fish gelatin to produce a crosslinked product of the cold-water fish gelatin is not particularly limited, and examples thereof include a method of applying heat (that is, a method of applying thermal energy), and a method of applying an active ray or radiation (for example, an electron beam) (that is, a method of applying light energy). Among them, a method of applying thermal energy (in other words, heating) (that is, a thermal crosslinking method) is preferred from the viewpoint of more easily obtaining a crosslinked product of the cold-water fish gelatin.

The method of thermally crosslinking the cold-water fish gelatin is not particularly limited, and typical examples thereof include a method of heating the cold-water fish gelatin in a reduced pressure environment at 100 to 200°C for 1 to 8 hours. In the above-mentioned method, for example, an amino group in the cold-water fish gelatin and other reactive groups (for example, a carboxy group and a mercapto group) undergo a reaction to produce a crosslinked product.

The crosslinked product of a cold-water fish gelatin may be obtained by reacting a cold-water fish gelatin with a crosslinking agent. The crosslinking agent is not particularly limited, and examples thereof include genipin, a polybasic acid activated with N-hydroxysuccinimide or N-sulfoxysuccinimide, an aldehyde compound, an acid anhydride, dithiocarbonate, and diisothiocyanate.

Examples of the usable crosslinking agent also include the compounds described in paragraphs [0021] to [0024] of WO 2018/079538 A1, the content of which is incorporated herein.

In the biological tissue adhesive sheet of the present embodiment, the thickness of the nonwoven fabric can be appropriately adjusted according to the intended use, application site, and the like of the biological tissue adhesive sheet. Specifically, for example, when the biological tissue adhesive sheet is applied to an organ such as a lung in respiratory surgery or gastroenterological surgery, the thickness of the nonwoven fabric is in the range of 100 µm or more and 500 µm or less. When the thickness of the nonwoven fabric is within the above-mentioned range, the biological tissue adhesive sheet has both excellent bulk strength and excellent flexibility.

When the thickness of the nonwoven fabric is 100 µm or more, the biological tissue adhesive sheet has better bulk strength. When the thickness of the nonwoven fabric is 500 µm or less, the biological tissue adhesive sheet has better flexibility.

### <Agent>

In one aspect, the biological tissue adhesive sheet of the present embodiment preferably contains an agent fixed to the nonwoven fabric. The term "fix" also encompasses to "encapsulate" and "adsorb" the agent.

The agent is selected according to the intended use, application site, and the like of the biological tissue adhesive sheet. Examples of the agent include, but are not limited to, an anticancer agent, an antimicrobial agent, and a growth factor. One kind of agent may be used alone, or two or more kinds thereof may be used in combination.

Examples of the anticancer agent include, but are not limited to, cisplatin, carboplatin, nedaplatin, and oxaliplatin. Note that the above-mentioned anticancer agents are classified into platinum complexes (platinum compounds), but the anticancer agent usable in the present invention is not limited thereto, and examples thereof may include: alkylating agents (such as nitrogen mustards (e.g., cyclophosphamide) and nitrosoureas (e.g., nimustine)); antimetabolites (such as antifolates (e.g., pemetrexed), pyrimidine metabolism inhibitors, purine metabolism inhibitors, and nucleotide analogs); topoisomerase inhibitors; microtubule inhibitors; antitumor antibiotics (such as doxorubicin); and molecular target agents.

Examples of the antimicrobial agent include antibacterial antibiotics and antifungal antibiotics. More specific examples include, but are not limited to, antimicrobial agents classified into penicillins (such as Penicillin G, ampicillin, and bacampicillin), cephems (such as first generation (e.g., cefazolin), second generation (e.g., cefotiam), third generation (e.g., cefdinir), and fourth generation (e.g., cefepime)), carbapenems, monobactams, and penems. In addition to the above-mentioned antimicrobial agents that are also comprehensively referred to as β-lactam antimicrobial agents, for example, aminoglycoside, lincomycin, chloramphenicol, macrolide, ketolide, polypeptide, glycopeptide, and tetracycline antimicrobial agents can also be used. Moreover, synthetic antimicrobial agents classified into quinolones, oxazolidinones, sulfa agents, and the like can also be used.

Examples of the growth factor include, but are not limited to, vascular endothelial growth factors (VEGFs), basic fibroblast growth factors (bFGFs), platelet-derived growth factors (PDGFs), hepatocyte growth factors (HGFs), and transforming growth factors-β (TGF-βs).

The agent may be subjected to a sustained release treatment by a conventionally known method as necessary. Owing to the sustained release treatment, when the agent is, for example, an anticancer agent, the effect of the anticancer agent on the target cancer cell can be further enhanced.

Here, as an application example of the biological tissue adhesive sheet of the present embodiment, treatment of malignant pleural mesothelioma will be described as an example.

Pleural mesothelioma causes, even in the case where it is possible to apply surgery (resection) by extrapleural pneumonectomy (EPP) or pleurectomy/decortication (P/D), postoperative recurrences in many cases, and is known to be one of malignant diseases having poor prognosis.

The biological tissue adhesive sheet of the present embodiment has excellent adhesiveness to surfaces of soft tissues including the pulmonary pleura in a wet environment, and contains, as the agent fixed to the nonwoven fabric, an anticancer agent such as cisplatin. Therefore, it is considered that the biological tissue adhesive sheet can release the anticancer agent sustainedly, and can kill cancer cells remaining after resection of a cancer lesion.

As described above, the biological tissue adhesive sheet of the present invention is capable of having excellent biocompatibility and tissue adhesiveness, and also having sustained agent releasability. Therefore, the biological tissue adhesive sheet can be applied not only to use for the purpose of reinforcing sutures after surgery, preventing air leakage, and the like as with conventional medical sheets, but also to use for the purpose of treating a biological tissue after surgery.

### [Method for Producing Biological Tissue Adhesive Sheet]

The method for producing a biological tissue adhesive sheet is not particularly limited, and typical examples thereof include a method of producing a nonwoven fabric by a conventionally known method such as electrospinning, melt blowing, or spunbonding using a cold-water fish gelatin, and then crosslinking the cold-water fish gelatin.

In particular, the following production method is preferred as the method for producing the present biological tissue adhesive sheet in that a biological tissue adhesive sheet having excellent uniformity is more easily obtained.

A method for producing a biological tissue adhesive sheet according to an embodiment of the present invention (hereinafter, the method is also referred to as "the present production method") includes:
preparing a composition by adding a cold-water fish gelatin and optionally a crosslinking agent and/or an agent to a solvent, the cold-water fish gelatin being at least one selected from the group consisting of cod gelatin, sea bream gelatin, salmon gelatin, and a genetically-engineered gelatin derived from cod, sea bream, or salmon,
electrospinning the composition to prepare a nonwoven fabric made of fibers containing the cold-water fish gelatin (hereinafter, the step is also referred to as an "electrospinning step"); and
applying energy to the nonwoven fabric to produce a biological tissue adhesive sheet (hereinafter, the step is also referred to as an "energy application step");
wherein the nonwoven fabric has a thickness in a range from 100 µm to 500 µm.

Hereinafter, the above-mentioned steps will be described.

### (Electrospinning Step)

The electrospinning step is a step of electrospinning a composition to prepare a nonwoven fabric.

In the electrospinning, a high voltage is applied to a composition containing the cold-water fish gelatin and a solvent so that the composition may be charged to form fibers, and the fibers are deposited to form a nonwoven fabric. As for a method for charging the composition, it is preferred to connect an electrode connected to a high-voltage power supply device to the composition itself or a container, and typically apply a voltage of 1 to 100 kV, and it is more preferred to apply a voltage of 5 to 50 kV.

The kind of voltage may be either direct current or alternating current.

The temperature during electrospinning is not particularly limited, and may be appropriately adjusted according to the boiling point and volatility of the solvent contained in the composition. In one embodiment, the temperature is preferably 10 to 30°C.

Since the present production method includes this step, the nonwoven fabric is produced without heating the composition, in other words, without heating the cold-water fish gelatin. Therefore, unintentional crosslinking of the cold-water fish gelatin is reduced, and a biological tissue adhesive sheet having a more uniform structure (more uniform fiber diameter or the like) is easily obtained.

The composition contains the cold-water fish gelatin, and a solvent. The content of the cold-water fish gelatin in the composition is not particularly limited, but from the viewpoint of obtaining a biological tissue adhesive sheet having an even better effect of the present invention, the content is generally preferably 5 to 50 mass/volume% (g/mL) with respect to the total volume-based amount (mL) of the solvent in the composition. The composition may contain one kind of cold-water fish gelatin alone or two or more kinds thereof. When the composition contains two or more kinds of cold-water fish gelatins, it is preferred that the total content of the cold-water fish gelatins be within the above-mentioned numerical range.

The solvent contained in the composition is not particularly limited, and a solvent capable of dissolving and/or dispersing the cold-water fish gelatin is preferred. Examples of the usable solvent include water, organic solvents, and mixtures thereof. Examples of the usable water include ultrapure water, deionized water, and distilled water. The organic solvent is not particularly limited, and alcohols having 1 to 3 carbon atoms, esters, and the like can be used. Ethanol is preferred.

The method for preparing the composition is not particularly limited, and a known method can be used. For example, the cold-water fish gelatin may be added to the solvent and heated as necessary.

The heating temperature in this case is preferably a temperature at which thermal crosslinking of the cold-water fish gelatin does not proceed. Specifically, the heating temperature is preferably 40 to 90°C.

The composition may contain components other than those described above. Examples of the components other than those described above include a crosslinking agent and an agent.

Depending on the intended use, application site, and the like of the biological tissue adhesive sheet, it is preferred that the composition do not substantially contain a crosslinking agent from the viewpoint that an unintended action by an unreacted crosslinking agent is less likely to occur.

### (Energy Application Step)

The energy application step is a step of applying energy to the nonwoven fabric to produce a biological tissue adhesive sheet. Owing to the application of energy, at least a part of the cold-water fish gelatin undergoes intermolecular and/or intramolecular crosslinking, and the resulting biological tissue adhesive sheet has even better bulk strength and even better water resistance.

The energy to be applied is not particularly limited, and may be light and/or heat. In particular, it is preferred that the energy be applied by heating the nonwoven fabric from the viewpoint of more easily obtaining the biological tissue adhesive sheet.

The heating method is not particularly limited, and typical examples thereof include a method of heating the nonwoven fabric in a reduced pressure environment at 100 to 200°C for 1 to 8 hours. More specifically, for example, when the raw material cold-water fish gelatin has a molecular weight (Mw) of about 100,000, the nonwoven fabric is heated for 1 to 6 hours in a reduced pressure environment at 140 to 160°C (for example, 150°C).

Furthermore, the present production method may include irradiating the biological tissue adhesive sheet obtained by the energy application step with ultraviolet rays to hydrophilize surfaces of the fibers containing the cold-water fish gelatin (the step is also referred to as an "ultraviolet irradiation step").

Hereinafter, the ultraviolet irradiation step will be described.

### (Ultraviolet Irradiation Step)

The ultraviolet irradiation step is a step of further irradiating the fibers containing the crosslinked cold-water fish gelatin (that is, cold-water fish gelatin fibers) with ultraviolet rays to hydrophilize surfaces of the fibers. The surface treatment by ultraviolet irradiation reduces the contact angle between the fibers and a water droplet, and the fibers are more easily swollen in the presence of moisture. Meanwhile, when the fibers are brought into contact with a tissue in a dry state, the fibers after ultraviolet irradiation still have excellent adhesiveness.

The conditions for ultraviolet irradiation are not particularly limited, and the fibers are usually irradiated for 0.5 minutes to 30 minutes, more preferably for 1 minute to 30 minutes, and still more preferably for 5 minutes to 30 minutes. When the ultraviolet irradiation time is within the above-mentioned range, the surfaces of the cold-water fish gelatin fibers can be appropriately hydrophilized without impairing the production efficiency. In addition, the degree of hydrophilization of the surfaces of the cold-water fish gelatin fibers can be adjusted by adjusting the ultraviolet irradiation time within the above-mentioned range.

The ultraviolet intensity is preferably 0.05 to 50 mW/cm², and more preferably 0.5 to 10 mW/cm². The integrated light quantity of ultraviolet rays is preferably 1 to 100 J/cm², and more preferably 5 to 100 J/cm².

The device for ultraviolet irradiation is not particularly limited, and a commercially available ultraviolet irradiation device may be used. Usually, it is sufficient to irradiate one surface (surface that comes into contact with a target tissue) of the biological tissue adhesive sheet with ultraviolet rays, but both surfaces of the sheet may be irradiated with ultraviolet rays. In this case, the surfaces to be irradiated (front and back surfaces of the sheet) are preferably switched at a certain time interval (for example, every several to several tens seconds, every 1 minutes, or every 5 minutes). In addition, since the fiber surface is hydrophilic after ultraviolet irradiation, it is preferred to store the sheet in a dry atmosphere such as in the presence of a dehumidifier.

### [Biological Tissue Reinforcement Material Kit]

The biological tissue reinforcement material kit according to an embodiment of the present invention includes the biological tissue adhesive sheet.

The kit of the present embodiment includes an instruction describing how to use the biological tissue adhesive sheet of the present invention, precautions for use, and the like.

As used herein, the term "instruction" encompasses a package insert, a package label, and a direction for use, and is not particularly limited. Examples of the instruction include packaging insertions, prescribing information, and leaflets. The instruction may be provided on a paper medium or may be provided in a form such as an electronic medium (for example, a website or an e-mail provided on the Internet).

Hereinafter, the present invention will be described in more detail with reference to examples. Materials, amounts used, proportions, contents of treatment, treatment procedures, and the like shown in the following examples can be appropriately changed without departing from the gist of the present invention. Therefore, the scope of the present invention should not be restrictively interpreted by the following examples.

### Examples

### [Preparation of Cold-Water Fish Gelatin Fiber Sheet]

To 5 mL of a 60% aqueous ethanol solution, 1.5 g of a gelatin derived from Alaska pollock (Mw = 33,000, manufactured by Nitta Gelatin Inc., hereinafter also referred to as a "cod gelatin") was added, and dissolved on a water bath set at 55°C. Then, the obtained gelatin solution was transferred to a syringe having a volume of 5 mL and equipped with an 18 gauge needle, and a nonwoven fabric made of cod gelatin fibers was prepared on a collector by electrospinning.

The obtained nonwoven fabric was heated (thermally crosslinked) in a reduced pressure environment at 150°C for 3 hours to produce a cod gelatin fiber sheet.

As a result of observation with a scanning electron microscope (SEM), it was recognized that the cod gelatin fibers had a fiber diameter of 2 to 5 µm, and that a fiber sheet having a uniform structure was obtained (Fig. 1). The sheet had a thickness of about 200 µm.

### [Burst Strength Test]

The cod gelatin fiber sheet obtained as described above was subjected to a burst strength test using a pleura separated from a porcine lung as an adherend.

Figs. 2(a) to 2(c) show an outline of the burst strength test. Fig. 2(a) is a photograph showing a configuration of an apparatus used in the test, Fig. 2(b) is a schematic view showing a cross section of a test chamber, and Fig. 2(c) is a schematic view showing a configuration of the adherend (porcine pleura) and the cod gelatin fiber sheet.

According to ASTM F2392-04 (Standard Test Method for Burst Strength of Surgical Sealants), a hole having a diameter of 3 mm was made in the center of the pleural tissue formed to have a diameter of 30 mm, and the cod gelatin fiber sheet formed to have a diameter of 15 mm was attached to the pleural tissue (Fig. 2(c)). The tissue and the sheet were left standing for 10 minutes, and then placed in the test chamber (Fig. 2(b)).

Physiological saline was flowed at a flow rate of 2 mL/min from the direction indicated by the arrows in Figs. 2(a) and 2(b), and the pressure when the cod gelatin fiber sheet attached to the pleural tissue was broken was measured by a pressure gauge (Fig. 2(a)).

As a comparative example, nonwoven fabrics (NEOVEIL (registered trademark) Type NV-M-015G (thickness: 150 µm), Type NV-M-03G (thickness: 300 µm), and Type NV-M-05G (thickness: 500 µm) manufactured by GUNZE LIMITED) made of polyglycolic acid were subjected to the above-mentioned burst strength test.

As a result, the cod gelatin fiber sheet produced in the example of the present invention had a burst strength of about 8.5 (cmH₂O) (n=3). On the other hand, as for the nonwoven fabrics of the comparative example, in all the cases where the thickness was 150 µm, 300 µm, or 500 µm, the value of burst strength was only about 1 to 2 (cmH₂O) (n = 3).

It should be noted here that for the cod gelatin fiber sheet produced in the example of the present invention, it was possible to measure the pressure when the sheet was broken without separation at the adhesion site between the pleural tissue and the sheet while flowing the physiological saline in the test chamber, whereas for the nonwoven fabrics of the comparative example, separation occurred at the adhesion site between the pleural tissue and the sheet by flowing the physiological saline (n = 5). More specifically, the burst strength shown for the nonwoven fabrics of the comparative example should be understood more accurately not as "a pressure value when the nonwoven fabric attached to the pleural tissue was broken" (that is, a value indicating the film strength as referred to herein), but as "a pressure value when the nonwoven fabric attached to the pleural tissue was separated from the adhesion site" (that is, the pressure value when the adhesiveness to the adherend (biological tissue) was lost).

Furthermore, cod gelatin fiber sheets having a thickness of about 250 µm, and about 400 µm prepared by the same procedure as described above were subjected to the burst strength test. As a result, it was recognized that these cod gelatin fiber sheets had a film strength equivalent to that of the above-described cod gelatin fiber sheet having a thickness of about 200 µm (n = 3).

From these results, it was confirmed that the cold-water fish fiber sheet of the present invention is superior in tissue adhesiveness and film strength to conventional medical sheets.

Moreover, cod gelatin fiber sheets, obtained by the same procedure as described above and by subjecting thermal crosslinking for 5 hours, were subjected to the burst strength test. As a result, it was recognized that these cod gelatin fiber sheets had a burst strength of about 20 (cmH₂O) and it was 2 times or more the film strength than that of the cod gelatin fiber sheets obtained with thermal crosslinking for 3 hours (n = 3).

From this result, it was confirmed that, in the case where a crosslinking process is performed by a thermal crosslinking method, the film strength of the cold-water fish gelatin fiber sheet can be changed in consideration of the kind and the molecular weight of the cold-water fish gelatin, and the thickness of the cold-water fish gelatin fiber sheet, and the like.

This suggests that, in the biological tissue adhesive sheet of the present invention, the film strength can be adjusted according to the intended use, application site, and the like of the sheet. In other words, in the biological tissue adhesive sheet of the present invention, even in the case where the composition of a fiber (cold-water fish gelatin fiber) constituting a nonwoven fabric is the same, the film strength of a sheet can be changed by changing the conditions of a crosslinking process. Therefore, it is possible to select a sheet having more preferable characteristics according to the intended use, application site, and the like of the sheet.

### <Example 2>

### [Preparation of Cold-Water Fish Gelatin Fiber Sheets under Different Thermal Crosslinking Conditions and Characteristic Evaluation of the Sheets]

Cod gelatin fiber sheets (thickness: about 200 µm) were prepared by the same procedure as in Example 1 except that the thermal crosslinking time was 1 hour or 5 hours.

The obtained cod gelatin fiber sheets were observed with a scanning electron microscope (SEM). As a result, both the fiber sheets had a fiber diameter of 1 to 3 µm and had a uniform structure.

### [Evaluation of Physical Properties]

On the surfaces of the cod gelatin fiber sheets obtained with thermal crosslinking for 5 hours in this example, 1 µL of pure water was dropped, and the contact angle of the water droplet after 5 seconds was measured. As a result, it was recognized that the contact angle of the water droplet was about 45°.

A test piece was cut out from the sheet, and the test piece was immersed in physiological saline for 60 minutes to evaluate the degree of swelling. As a result, it was recognized that swelling ratio was about 10%.

The sheet molded into a dumbbell shape in accordance with JIS K 7127: 1999 was subjected to a tensile test. As a result, it was recognized that the Young's modulus was about 1.7 KPa and the tensile strength was about 0.11 MPa.

These physical properties are considered to reflect the crosslinking density in the cod gelatin fiber sheet described above or the structural change of the gelatin due to heat treatment. Therefore, it is presumed that these physical properties tend to vary depending on the thickness of the cod gelatin fiber sheet, the thermal crosslinking conditions (thermal crosslinking time), and the like.

In addition, cod gelatin fiber sheets obtained with thermal crosslinking for 1 hour or 5 hours were subjected to the burst strength test. As a result, it was recognized that the cod gelatin fiber sheets had a film strength equivalent to that of the cod gelatin fiber sheet obtained with thermal crosslinking for 3 hours in Example 1 (n = 3).

### [Burst Strength Test on Lungs Isolated from Rat]

The seam bream gelatin fiber sheet obtained with thermal crosslinking for 5 hours in this example and the nonwoven fabric (thickness: 150 µm) of the comparative example that is made of polyglycolic acid (hereinafter, the nonwoven fabric is also referred to as a "PGA sheet") were subjected to a burst strength test using lungs isolated from a rat as an adherend.

Specifically, both lungs including the trachea were isolated from a rat, a defect having a depth of 2 mm was produced in the pleura of the isolated lungs with a 20 G injection needle, and a sheet piece having a diameter of about 7 mm and a thickness of 0.2 mm was attached to cover the defect.

Then, the isolated lungs to which the sheet piece was attached were left standing for 10 minutes, and air was sent from the trachea at a flow rate of 1.0 mL/sec in a state in which the lungs were immersed in physiological saline at 37°C. The pressure when the sheet piece attached to the defect was broken and air leakage occurred due to expansion of the lungs was measured by a pressure gauge.

As a result, the burst strength in the case of using the cod fiber sheet was about 20 (cmH₂O) which was significantly higher than the burst strength (about 15 (cmH₂O)) in the case of using the nonwoven fabric made of polyglycolic acid (that is, the PGA sheet).

### [Toxicity Test on Cells]

The seam bream gelatin fiber sheet obtained with thermal crosslinking for 5 hours in this example was subjected to a toxicity test on L-929 cells.

More specifically, L-929 cells were cultured on a sheet for 24 hours, and then the number of cells was counted by a WST-8 assay. As for the morphology of the cells, actin staining was performed using DAPI (a nucleic acid fluorescence staining reagent that is impermeable to cell membranes), and then the immobilized cells were observed with a fluorescence microscope.

As a result, it was recognized that 90% or more of the cultured L-929 cells were alive for the cod gelatin fiber sheet.

### [Biocompatibility/Degradability Test]

The cod gelatin fiber sheet prepared with thermal crosslinking for 5 hours in this example and the nonwoven fabric made of polyglycolic acid (that is, the PGA sheet) were subjected to a biocompatibility test and a degradability test by subcutaneous implantation in the back of rats.

More specifically, the center of the back of each rat was shaved and disinfected, then the skin was incised, a sheet having a diameter of about 7 mm and a thickness of 0.2 mm was implanted into the back, and then the skin was sutured.

The observation period was set to 3 days, 7 days, 14 days, and 21 days after surgery, and the implanted sheet and the surrounding tissue were collected in each observation period and subjected to optical microscope observation and tissue observation by hematoxylin-eosin staining.

As a result, in the rat implanted with the cod gelatin fiber sheet, the sheet was completely degraded on day 21 after surgery, and no strong inflammatory reaction was observed. More specifically, on day 14 after surgery, healing of the incision site and degradation of the sheet proceeded to such an extent that the incision site and the sheet could hardly be observed with the naked eye. From these results, it is considered that the degradation reaction of the cod gelatin fiber sheet proceeded by the enzyme secreted from the tissue cells around the sheet as the skin incised at the time of sheet implantation healed.

On the other hand, in the rat implanted with the nonwoven fabric made of polyglycolic acid (that is, the PGA sheet), the sheet remained even on day 21 after surgery. This is considered to be because the degradation of the PGA sheet is a hydrolysis reaction, and thus the degradation speed of the sheet was slower than the progress of the healing of the incision site. In such a case, there is a concern about the possibility of continuation of an inflammatory reaction due to the sheet remaining after tissue healing.

### <Example 3>

A cod gelatin fiber sheet was prepared by the same procedure as in Example 1 except that the thermal crosslinking time was 5 hours. Hereinafter, the cod gelatin fiber sheet thus obtained is also referred to as "AdFS". In a UV irradiation box (produced by the National Institute for Materials Science), the obtained cod gelatin fiber sheet was left standing and irradiated with ultraviolet rays (source: a UV lamp, manufactured by MIYATA ELEVAM Inc.) of 185 nm and 254 nm at normal temperature for 60 minutes to surface-treat the fibers in the fiber sheet. Hereinafter, the cod gelatin fiber sheet obtained in this example, which was subjected to UV irradiation for 60 minutes, is also referred to as "UV-AdFS".

As for UV-AdFS, an absorption spectrum was measured by Fourier transform infrared spectroscopy (FT-IR), and the molecular structure was analyzed. As a result, an absorption around 3280 cm⁻¹ derived from -NH-stretching vibration, an absorption around 2925 to 2970 cm⁻¹ derived from -CH₂-stretching vibration and an absorption around 2875 cm⁻¹ derived from -CH₃-stretching vibration of an alkyl terminal were observed.

As a result of observation with a scanning electron microscope (SEM), it was recognized that UV-AdFS had a fiber diameter in the range of 1 to 2 µm, and that fiber sheet having a uniform structure was obtained.

### [Evaluation of Physical Properties]

On the surfaces of AdFS and UV-AdFS, 1 µL of pure water was dropped, and the contact angle of the water droplet after 1 second was measured (n = 3).

As a result, in AdFS, the contact angle of the water droplet was about 40°, and in UV-dFS, the contact angle of the water droplet was about 0°. As for cod gelatin fiber sheets produced by setting the ultraviolet irradiation time for AdFS to 5 minutes, 10 minutes or 30 minutes, the measurement results of the contact angle were about 15°, 7.5° and about 0°, respectively. From these results, it was recognized that in the fiber sheet containing the cod gelatin, the contact angle of the water droplet is greatly reduced as compared with the case of no irradiation, and the surfaces of the cod gelatin fibers become highly hydrophilic by ultraviolet irradiation for 10 minutes or less, and further, the surfaces of the cod gelatin fibers become almost completely hydrophilic by irradiation for 30 minutes.

A test piece was cut out from each of AdFS and UV-AdFS, and the test piece was immersed in physiological saline at 37°C for 24 hours to evaluate the degree of swelling. As a result, both the sheets showed about the same swelling ratio in the range of about 12.5% to about 14%.

In addition, the fiber sheets molded into a dumbbell shape in accordance with JIS K 7127: 1999 were subjected to a tensile test. As a result, almost the same stress-strain curves were obtained for AdFS and UV-AdFS.

These physical properties are considered to reflect the crosslinking density in the cod gelatin fiber sheet described above or the structural change of the gelatin due to heat treatment. Therefore, it is presumed that these physical properties tend to vary depending on the thickness of the cold-water fish gelatin fiber sheet, the thermal crosslinking conditions (thermal crosslinking time), and the like.

### [Burst Strength Test on Porcine Pleura]

Cod gelatin fiber sheet prepared with thermal crosslinking for 1 hour (hereinafter also referred to as "AdFS1") and a cod gelatin fiber sheet obtained by subjecting AdFS1 to ultraviolet irradiation for 60 minutes (hereinafter also referred to as "UV-AdFS1") were subjected to the burst strength test on a porcine pleura with the standing time after attaching the sheets to the pleural tissue set to 0.5 minutes, 1 minute, 2 minutes, 5 minutes, or 10 minutes.

The results are shown in Fig. 3.

Fig. 3 is a graph showing a relation between the standing time (min) and the burst strength (cmH₂O) of AdFS1 and UV-AdFS1 in the burst strength test on the porcine pleura.

As shown in Fig. 3, in the case of UV-AdFS1 (filled circles), the burst strength reached the maximum value in a standing time shorter than 10 minutes (more specifically, in about 5 minutes), whereas in the case of AdFS1 (open circles) not irradiated with ultraviolet rays, the burst strength was the highest when the standing time was 10 minutes.

These results suggest that the hydrophilization of the surfaces of the cod gelatin fiber sheets by ultraviolet irradiation increased the moisture absorption speed of the cod gelatin fiber sheets from the pleural tissue, resulting in an improvement in the speed of adhesion between the cod gelatin fiber sheets and the pleural tissue.

In the same manner as described above with respect to the burst strength test on the porcine pleura using the cod gelatin fiber sheet of Example 1, also in the case of UV-AdFS1, it was possible to measure the pressure when the sheet was broken without separation at the adhesion site between the pleural tissue and the sheet while flowing physiological saline in the test chamber.

### [Degradability Test Using Collagenase]

UV-AdFS was subjected to an enzymatic degradability test using a solution obtained by dissolving a collagenase in physiological saline (that is, a collagenase solution).

Specifically, a sample (UV-AdFS) was placed in a container containing the collagenase solution, and immersed in the collagenase solution for 30 minutes, 60 minutes, 90 minutes, 120 minutes, 150 minutes, and 180 minutes. Then, the collagenase solution was removed, and the weight of the remaining sample was measured to calculate the residual rate (%) with respect to the weight of the sample before being immersed in the collagenase solution.

As a result, after 120 minutes from the start of immersion in the collagenase solution, the residual weight of UV-AdFS was about 20% of the initial sheet weight, the residual weight was about 10% after 150 minutes, and the sample was almost completely degraded after 180 minutes (n = 3).

### [Toxicity Test on Cells]

UV-AdFS was subjected to a toxicity test on L-929 cells.

More specifically, L-929 cells were cultured on UV-AdFS for 24 hours, and then the number of cells was counted by a WST-8 assay. As for the morphology of the cells, actin staining was performed using DAPI (a nucleic acid fluorescence staining reagent that is impermeable to cell membranes), and then the immobilized cells were observed with a fluorescence microscope.

As a control, the number of cells was counted by the WST-8 assay using cells obtained by culturing L-929 cells in a normal culture medium for 24 hours.

As a result, it was recognized that 90% or more of the cultured L-929 cells were alive for UV-AdFS.

In addition, in the case of UV-AdFS, the proliferation rate after 24 hours of culture was about 300%, which was significantly higher than those of the control (about 220%).

### Industrial Applicability

Since the biological tissue adhesive sheet of the present invention has tissue adhesiveness superior to that of conventional medical sheets and also has excellent film strength, the sheet is expected to be applied to medical fields such as respiratory surgery, gastroenterological surgery, and gastroenterological medicine.

In addition, when the biological tissue adhesive sheet of the present invention contains an agent (for example, an anticancer agent) fixed to the nonwoven fabric made of fibers containing a crosslinked cold-water fish gelatin, the sheet can be used not only as a tissue reinforcement material but also as an agent eluting sheet.

## Claims

1. A biological tissue adhesive sheet comprising a nonwoven fabric made of fibers containing a crosslinked cold-water fish gelatin,
wherein the cold-water fish gelatin is at least one selected from the group consisting of cod gelatin, sea bream gelatin, salmon gelatin, and a genetically-engineered gelatin derived from cod, sea bream, or salmon, and
wherein the nonwoven fabric has a thickness in a range from 100 µm to 500 µm.

2. The biological tissue adhesive sheet as claimed in claim 1, wherein the cold-water fish gelatin is cod gelatin, and wherein the contact angle of a water droplet is 15° or less, the contact angle of the water droplet being measured after 1 second after dropping 1 µL of pure water on the surfaces of the biological tissue adhesive sheet.

3. The biological tissue adhesive sheet as claimed in claim 2, wherein the contact angle of the water droplet is 0°.

4. The biological tissue adhesive sheet as claimed in any one of claims 1 to 3, comprising an agent fixed to the nonwoven fabric.

5. The biological tissue adhesive sheet as claimed in claim 4, wherein the agent is an anticancer agent.

6. The biological tissue adhesive sheet as claimed in claim 4, wherein the agent is an antimicrobial agent.

7. The biological tissue adhesive sheet as claimed in claim 4, wherein the agent is a growth factor.

8. A biological tissue reinforcement material kit comprising the biological tissue adhesive sheet as claimed in any one of claims 1 to 7, and an instruction describing how to use the biological tissue adhesive sheet and/or precautions for use.

9. A method for producing a biological tissue adhesive sheet, the method comprising:
preparing a composition by adding a cold-water fish gelatin and optionally a crosslinking agent and/or an agent to a solvent, the cold-water fish gelatin being at least one selected from the group consisting of cod gelatin, sea bream gelatin, salmon gelatin, and a genetically-engineered gelatin derived from cod, sea bream, or salmon,
electrospinning the composition to prepare a nonwoven fabric made of fibers containing the cold-water fish gelatin; and
applying energy to the nonwoven fabric to produce a biological tissue adhesive sheet;
wherein the nonwoven fabric has a thickness in a range from 100 µm to 500 µm.

10. The method as claimed in claim 9, wherein the agent is at least one selected from the group consisting of an anticancer agent, an antimicrobial agent, and a growth factor.

11. The method as claimed in claim 9 or 10, wherein
in the preparing step, the cold-water fish gelatin is added to a solvent, and
in the electrospinning step, a nonwoven fabric made of fibers consisting of the cold-water fish gelatin is prepared.

12. The method as claimed in any one of claims 9-11, further comprising irradiating the biological tissue adhesive sheet with an ultraviolet ray to hydrophilize surfaces of the fibers containing the cold-water fish gelatin.

13. The method as claimed in claim 12, wherein the biological tissue adhesive sheet is irradiated with the ultraviolet ray for 0.5 minutes to 30 minutes.

## Patentansprüche

1. Klebefolie für biologisches Gewebe, die einen aus Fasern hergestellten Vliesstoff umfasst, die eine vernetzte Kaltwasserfischgelatine enthalten,
wobei die Kaltwasserfischgelatine mindestens eine ist, ausgewählt aus der Gruppe bestehend aus Kabeljaugelatine, Seebrassengelatine, Lachsgelatine und einer gentechnisch veränderten Gelatine, die aus Kabeljau, Seebrasse oder Lachs gewonnen wird, und
wobei der Vliesstoff eine Dicke in einem Bereich von 100 µm bis 500 µm aufweist.

2. Klebefolie für biologisches Gewebe nach Anspruch 1, wobei die Kaltwasserfischgelatine Kabeljaugelatine ist und wobei der Kontaktwinkel eines Wassertropfens 15° oder weniger beträgt, wobei der Kontaktwinkel des Wassertropfens 1 Sekunde nach dem Tropfen von 1 µl reinem Wasser auf die Oberflächen der Klebefolie für biologisches Gewebe gemessen wird.

3. Klebefolie für biologisches Gewebe nach Anspruch 2, wobei der Kontaktwinkel des Wassertropfens 0° beträgt.

4. Klebefolie für biologisches Gewebe nach einem der Ansprüche 1 bis 3, die ein Mittel umfasst, das an dem Vliesstoff fixiert ist.

5. Klebefolie für biologisches Gewebe nach Anspruch 4, wobei das Mittel ein Antikrebsmittel ist.

6. Klebefolie für biologisches Gewebe nach Anspruch 4, wobei das Mittel ein antimikrobielles Mittel ist.

7. Klebefolie für biologisches Gewebe nach Anspruch 4, wobei das Mittel ein Wachstumsfaktor ist.

8. Kit mit Verstärkungsmaterial für biologisches Gewebe, das die Klebefolie für biologisches Gewebe nach einem der Ansprüche 1 bis 7 und eine Anweisung, die beschreibt, wie die Klebefolie für biologisches Gewebe zu verwenden ist und/oder Vorsichtsmaßnahmen für die Verwendung umfasst.

9. Verfahren zum Herstellen einer Klebefolie für biologisches Gewebe, wobei das Verfahren umfasst:
Vorbereiten einer Zusammensetzung durch Zugeben einer Kaltwasserfischgelatine und optional eines Vernetzungsmittels und/oder eines Mittels zu einem Lösungsmittel, wobei die Kaltwasserfischgelatine mindestens eine ist, ausgewählt aus der Gruppe bestehend aus Kabeljaugelatine, Seebrassengelatine, Lachsgelatine und einer gentechnisch veränderten Gelatine, die aus Kabeljau, Seebrasse oder Lachs gewonnen wird,
Elektrospinnen der Zusammensetzung, um einen Vliesstoff aus Fasern vorzubereiten, die die Kaltwasserfischgelatine enthalten; und
Anwenden von Energie an den Vliesstoff, um eine Klebefolie für biologisches Gewebe herzustellen;
wobei der Vliesstoff eine Dicke in einem Bereich von 100 µm bis 500 µm aufweist.

10. Verfahren nach Anspruch 9, wobei das Mittel mindestens eines ist, ausgewählt aus der Gruppe bestehend aus einem Antikrebsmittel, einem antimikrobiellen Mittel und einem Wachstumsfaktor.

11. Verfahren nach Anspruch 9 oder 10, wobei
im Vorbereitungsschritt die Kaltwasserfischgelatine einem Lösungsmittel zugegeben wird, und
im Elektrospinnschritt ein Vliesstoff aus Fasern vorbereitet wird, die aus der Kaltwasserfischgelatine bestehen.

12. Verfahren nach einem der Ansprüche 9-11, weiter Bestrahlen der Klebefolie für biologisches Gewebe mit einer Ultraviolettstrahlung umfassend, um Oberflächen der Fasern, die die Kaltwasserfischgelatine enthalten, zu hydrophilisieren.

13. Verfahren nach Anspruch 12, wobei die Klebefolie für biologisches Gewebe 0,5 Minuten bis 30 Minuten lang mit der Ultraviolettstrahlung bestrahlt wird.

## Revendications

1. Feuille adhésive de tissu biologique comprenant un tissu non tissé constitué de fibres contenant une gélatine de poisson d'eau froide réticulée,
dans laquelle la gélatine de poisson d'eau froide est au moins une gélatine choisie dans le groupe consistant en la gélatine de cabillaud, la gélatine de daurade, la gélatine de saumon et une gélatine génétiquement modifiée dérivée de cabillaud, de daurade ou de saumon, et
dans laquelle le tissu non tissé présente une épaisseur comprise dans une plage de 100 µm à 500 µm.

2. Feuille adhésive de tissu biologique selon la revendication 1, dans laquelle la gélatine de poisson d'eau froide est de la gélatine de cabillaud, et dans laquelle l'angle de contact d'une goutte d'eau est inférieur ou égal à 15°, l'angle de contact de la goutte d'eau étant mesuré après 1 seconde après avoir déposé 1 µL d'eau pure sur les surfaces de la feuille adhésive de tissu biologique.

3. Feuille adhésive de tissu biologique selon la revendication 2, dans laquelle l'angle de contact de la goutte d'eau est de 0°.

4. Feuille adhésive de tissu biologique selon l'une quelconque des revendications 1 à 3, comprenant un agent fixé au tissu non tissé.

5. Feuille adhésive de tissu biologique selon la revendication 4, dans laquelle l'agent est un agent anticancéreux.

6. Feuille adhésive de tissu biologique selon la revendication 4, dans laquelle l'agent est un agent antimicrobien.

7. Feuille adhésive de tissu biologique selon la revendication 4, dans laquelle l'agent est un facteur de croissance.

8. Kit de matériau de renforcement de tissu biologique comprenant la feuille adhésive de tissu biologique selon l'une quelconque des revendications 1 à 7, et une instruction décrivant comment utiliser la feuille adhésive de tissu biologique et/ou les précautions d'emploi.

9. Procédé de production d'une feuille adhésive de tissu biologique, le procédé comprenant :
la préparation d'une composition par ajout d'une gélatine de poisson d'eau froide et éventuellement d'un agent de réticulation et/ou d'un agent à un solvant, la gélatine de poisson d'eau froide étant au moins une gélatine choisie dans le groupe consistant en la gélatine de cabillaud, la gélatine de daurade, la gélatine de saumon et une gélatine génétiquement modifiée dérivée de cabillaud, de daurade ou de saumon,
l'électrofilage de la composition pour préparer un tissu non tissé constitué de fibres contenant de la gélatine de poisson d'eau froide ; et
l'application d'énergie au tissu non tissé pour produire une feuille adhésive de tissu biologique ;
dans lequel le tissu non tissé présente une épaisseur comprise dans une plage de 100 µm à 500 µm.

10. Procédé selon la revendication 9, dans lequel l'agent est au moins un agent choisi dans le groupe consistant en un agent anticancéreux, un agent antimicrobien et un facteur de croissance.

11. Procédé selon la revendication 9 ou 10, dans lequel :
dans l'étape de préparation, la gélatine de poisson d'eau froide est ajoutée à un solvant, et
dans l'étape d'électrofilage, un tissu non tissé constitué de fibres consistant en de la gélatine de poisson d'eau froide est préparé.

12. Procédé selon l'une quelconque des revendications 9-11, comprenant en outre l'exposition de la feuille adhésive de tissu biologique à un rayon ultraviolet pour hydrophiliser les surfaces des fibres contenant la gélatine de poisson d'eau froide.

13. Procédé selon la revendication 12, dans lequel la feuille adhésive de tissu biologique est exposée au rayon ultraviolet pendant 0,5 minute à 30 minutes.
